# EUROPEAN PATENT SPECIFICATION

(11) **EP 1 086 071 B1**
(45) Date of publication and mention of the grant of the patent: **19.10.2005**
(21) Application number: 99926516.8
(22) Date of filing: 04.06.1999
(51) Int. Cl.: C07C 49/653, C07C 49/753, A61K 35/78

(54) **HYPERFORIN DERIVATIVES, THE USE THEREOF AND FORMULATIONS CONTAINING THEM**
HYPERFORIN-DERIVATE, IHRE VERWENDUNG SOWIE DIESE ENTHALTENDE ZUBEREITUNGEN
DERIVES D'HYPERFORINE, LEUR UTILISATION ET FORMULATIONS LES CONTENANT

(30) Priority: 10.06.1998 IT MI981312
(43) Date of publication of application: 28.03.2001
(60) Divisional application: 05015870.8
(73) Proprietor: INDENA S.p.A., 20139 Milano (IT)
(72) Inventor: BOMBARDELLI, Ezio, I-20141 Milano (IT); MARAZZONI, Paolo, I-20139 Milano (IT)
(74) Representative: Minoja, Fabrizio
(86) International application number: PCT/EP1999/003880
(87) International publication number: WO 1999/064388

(56) References cited:
- EP-A- 0 599 307
- WO-A-97/13489
- BRONDZ, ILIA ET AL: "The relative stereochemistry of hyperforin" TETRAHEDRON LETTERS, vol. 23, no. 12, 1982, pages 1299-1300, XP002117275 Great Britain
- BRONDZ I. ET AL: 'The absolute configuration of hyperforin, an antibiotic from Hypericum perforatum L., based on the crystal structure determination of its p-bromobenzoate ester' ACTA CHEMICA SCANDINAVICA vol. 37, no. 3, 1983, pages 263 - 265
- BYSTROV N.S. ET AL: 'Chemistry of hyperforin. VI. General chemical characteristics' PLENUM PUBLISHING CORPORATION (TRANSL. OF BIOORG. KHIM. 1978, 4(6), PAGES 791-979) 1979, pages 575 - 580

## Description

The present invention relates to novel hyperforin derivatives, the use thereof for the treatment of depression and anxiety, as well as the formulations containing them.

Flowering tops of Hypericum perforatum contain a number of classes of structurally different substances acting directly or indirectly on central nervous system.

More particularly, said compounds comprise hypericin, hyperforin, and dimeric flavones which exert antidepressive and anxiolytic activities on animals and humans.

The action mechanisms of these compounds are different: anti-MAO action, action on serotonin release, and benzodiazepine-like activity.

Hyperforin, which is one of the main components of the lipophilic fraction of Hypericum perforatum flowering tops, has recently been the object of numerous studies which made it possible to establish its important role as antidepressant; studies carried out by the Applicant proved that this molecule has serotonin-mimetic activity.

Hyperforin is not very stable in the usual conditions of extraction and conservation; according to WO 97/13489 (Schwabe), the hyperforin content in a water-alcoholic extract of St.-John's-wort falls nearly to zero already after a few weeks.

Again according to WO 97/13489, in order to obtain stable extracts with a constant content in hyperforin, extraction, purification and conservation should be carried out in the presence of antioxidants such as vitamin C and the esters thereof, sulfated amino acids, etc.

The high instability of hyperforin makes therefore the preparation of formulations rather difficult.

Now hyperforin derivatives have been found which are stable and more active as antidepressants in specific pharmacological tests. The derivatives of the invention have the following formula I: 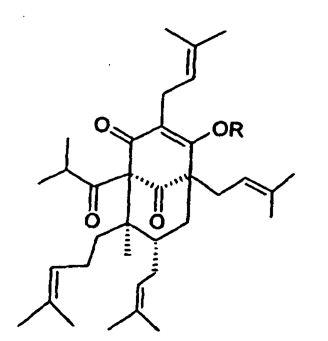 in which R is an aromatic acyl residue in which the aromatic moiety optionally carries one or more substituents, which can be the same or different, selected from hydroxy, methoxy, amino groups.

"Aromatic acyl residue" preferably means a benzoyl or cinnamyl residue having one or more amino or alkoxy groups.

Preferred R groups are p-aminobenzoyl, trimethoxybenzoyl, trimethoxycinnamoyl.

The hyperforin derivatives of the invention can be prepared with conventional methods for the acylation of hydroxy groups.

For example, hyperforin substantially pure or an extract enriched in hyperforin can be subjected to reaction with acid chlorides or anhydrides of RCOOH acids (R as defined above) in suitable solvents, such as pyridine.

According to a particularly convenient aspect of the invention, compounds I are prepared by extracting Hypericum perforatum flowering tops with carbon dioxide in supercritical conditions, subsequently partitioning between solvents and derivatizing hyperforin in the resulting extract.

Leaves and flowering tops of St.-John's-wort, separately or in mixture, mainly as natural mixture, are extracted with carbon dioxide in supercritical conditions under pressures ranging from 180 to 260 bars, preferably 240 bars and at temperatures ranging from 35 to 50°C, preferably 40°C. A lipophilic extract is obtained containing about 50% of hyperforin. The extract contains considerable amounts of xanthones, waxes, fatty acids and triglycerids. The Hyperforin percentage is subsequently increased, according to a further aspect of the invention, solubilizing the resulting extract in methanol or in partially aqueous acetonitrile and extracting then the solution with n-hexane or aliphatic hydrocarbons. The hydrocarbon phase contains undesired substances which are removed; the hydrophilic phase is diluted with about an equal volume of water and with aliphatic hydrocarbons. The extract of St.-John's-wort obtained by concentration of the lipophilic phase can be used for the preparation of the derivatives as described above.

The derivatives of the invention exert no activity *in vitro* on receptors, while being particularly active *in vivo,* exerting a dose-related strong antidepressive activity.

In an *in vivo* test in mice and rats, the compounds of the invention have shown a higher activity than hyperforin and Hypericum ethanolic or methanolic extracts.

As *in vivo* test to verify the antidepressive effect, were selected the escape deficit development test and the inhibition of the ethanol consumption in Sardinia alcohol preferring rats according to models known in literature.

In the escape deficit development test, the compounds of the invention have shown a higher activity than the known extracts and an activity comparable with that of known medicaments such as imipramine. In this test, rats are fastened and subjected to mild, short, unavoidable electric shocks for 50 min (pre-test). Twenty-four hours later, animals are tested for their ability to avoid the same stimuli on their tails, in a situation in which escape is impossible. A rat on the average makes 26 escapes out of 30 stimuli (naive controls), whereas an animal subjected to pre-test only makes 1-3 escapes (ED controls). Hyporeactivity induced by the pre-test does not take place in rats pre-treated for 1-3 weeks with antidepressants such as imipramine or fluoxetine. The compounds of the invention administered to rats one hour before exposure to the unavoidable stress cause an increase in reactivity to the escape test, which is enhanced when pre-treatment is effected for 1-2 weeks.

For example, treatment of rats with hyperforin acetate yields the results reported in the following Table:

**Table -**

| Antidepressive effect of Hyperforin acetate in rats in the escape test with a 2 week- pre-test. | | |
|---|---|---|
| Substances | Dose mg/kg | Number of escapes |
| Hyperforin acetate | 6.25 | 12.6 ± 2.8 |
| Hyperforin acetate | 12.5 | 17.3 ± 1.9 |
| Hyperforin acetate | 25.0 | 21.2 ± 1.3 |
| Hyperico alcoholic extract | 1000 | 15.6 ± 2.4 |
| Hyperico hexane extract | 600 | 16.9 ± 1.2 |
| Ed controls | | 2.6 ± 0.7 |
| Naive controls | | 23.6 ± 1.2 |

| | |
|---|---|
| Statistical analysis: | Kruskal-Wallis non parametric |
| ANOVA KW = 13.462 p = 0.0012 | p<0.01 |
| Hypericum alcoholic extract and hexane extract vs naive | p<0.01 |
| Hyperforin acetate 25 mg vs naive | n.s. |
| Naive vs AND | p<0.01 |

In the test of the reduction of alcohol consumption in Sardinia rats (which is an index of depression and anxiety) according to procedures known in literature, the products of the invention induce, after two-day administration, a 60 to 75% decrease in alcohol consumption in favour of water compared with controls.

The compounds of formula I can be formulated in soft-gelatin capsules, hard-gelatin capsules, tablets, suppositories; preferably the compounds of the invention are formulated in soft-gelatin capsules or in controlled-release formulations. The dosages of compounds in the formulations range from 5 to 50 mg per dose in the usual formulations and up to 200 mg in the controlled-release formulations, in this case the preferred dose being 200 mg per dose daily.

The examples reported hereinbelow illustrate the invention in greater detail.

### Example 1 - Preparation of a Hyperforin-enriched extract.

10 kg of biomass of *Hypericum perforatum* are extracted according to the procedure reported below, in a 25 L extraction plant for supercritical gas, equipped with two separators.

10 kg of *Hypericum perforatum* flowering tops mechanically dried, after collecting, at a temperature not above 60°C, are extruded into cubes so as to break cells and extracted with CO₂ in supercritical conditions under the.following experimental conditions:
- temperature: 45°C in the extractor, 30°C in the first separator and 20°C in the second separator;
- pressure: 240 bars in the extractor, 100 bars in the first separator and 50 bars in the second separator.

The CO₂ flow was 10 L per minute for 45 minutes. The extract was concentrated in the second separator, whereas most water present in the vegetable matrix was concentrated in the first separator. The extract present in the second separator is solubilised in 3.2 L of methanol and this solution is extracted with 3 x 1.5 L of n-hexane.

The hexane phase is counter-washed with 98% methanol using hyperforin as marker which should remain in the methanol phase. The hexane phase is removed whereas the combined metabolic ones are diluted with 0.6 L of water and re-extracted with 2 x 0.6 L of n-hexane.

The combined hexane phases are decolourized with 0.3% charcoal, dried over Na₂SO₄, then concentrated under vacuum at a temperature not above 40°C to an oil. 0.22 kg of a waxy extract are obtained, having an about 70% hyperforin content.

### Example 2 - Synthesis of hyperforin 3,4,5-trimethoxybenzoate

A solution of plant extract of Example 1 (1.0 g) in pyridine (4 mL) is added with 3,4,5-trimethoxybehzoyl chloride (323 mg), and the solution is stirred at room temperature for 24 hours. The reaction cannot be checked by TLC, as the starting material and the product have a very close Rf value in different solvents. The reaction mixture is diluted with water and extracted with an ether-hexane mixture (3:1). The organic phase is washed with diluted HCl, saturated NaHCO₃ (washing with saline solution causes an emulsion to form). After drying (Na₂SO₄) and evaporation, the residue is purified by chromatography on a silica gel column (ca 5 g), eluting first with petroleum ether to remove fats, then with hexane-EtOAc 95:5 to obtain hyperforin trimethoxybenzoate (317 mg) as a colourless oil.
C₄₅H₆₂O₈, MW 730
CI-MS:731 (M + H)+
IR (liquid film): 1732, 1660, 1634, 1589, 1465, 1331, 1153, 1130. 914 cm⁻¹
¹H NMR (200 MHz, CDCl₃): 7.27 (s, 2H), 5.04 (br S, 2H), 5.02 (br s, 2H), 3.86 (s, OMe), 3.82 (s, 2 x OMe), 3.10 (dd, J=15, 7 Hz, 1H), 2.92 (dd, J=15, 7 Hz, 1H), 1.66-1.53 (br s, 8 x 3H), 1.13 (d, J=6,S Hz, 3H), 1.04 (s, 3H), 0.99 (d, J=6.5 Hz, 3H).

### Example 3- Coated tablets (not according to the invention)

| | |
|---|---|
| Hyperforin acetate | 100 mg |
| Soy polysaccharides | 18.25 mg |
| Cross-linked sodium carboxymethylcellulose | 13.50 mg |
| Silica | 6.50 mg |
| Polyvinylpyrrolidone | 5.00 mg |
| Magnesium stearate | 0.50 mg |
| Coating: | |
| Hydroxypropyl methylcellulose | 3.75 mg |
| Talc | 2.75 mg |
| Titanium dioxide | 1.25 mg |
| Triacetin | 0.75 mg |
| Polysorbate 80 | 0.25 mg |
| Red iron oxide | 1.00 mg |

## Claims

1. Compounds of formula I: 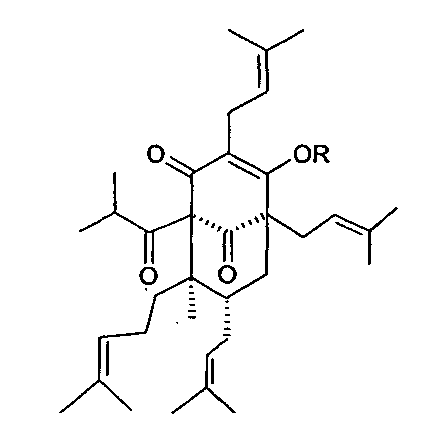 where R is an aromatic acyl residue in which the aromatic moiety optionally has one or more substituents, which can be the same or different, selected from hydroxy, methoxy, amino groups.

2. Compounds as claimed in claim 1 wherein R is selected from p-aminobenzoyl, trimethoxybenzoyl and trimethoxycinnamoyl.

3. A compound according to claim 2, wherein R is trimethoxybenzoyl.

4. Pharmaceutical compositions containing as active ingredient a compound of claims 1-3.

5. The use of compounds of formula I according to claim 1 for the preparation of an antidepressive medicament.

## Patentansprüche

1. Verbindungen der Formel I: 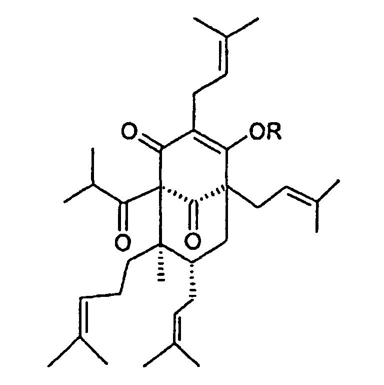 worin R einen aromatischen Acylrest darstellt, worin die aromatische Einheit gegebenenfalls einen oder mehrere Substituenten aufweist, die gleich oder verschieden sein können, ausgewählt aus Hydroxy-, Methoxy-, Aminogruppen.

2. Verbindungen nach Anspruch 1, worin R ausgewählt ist aus p-Aminobenzoyl, Trimethoxybenzoyl und Trimethoxycinnamoyl.

3. Verbindung nach Anspruch 2, worin R Trimethoxybenzoyl darstellt.

4. Pharmazeutische Zusammensetzungen, die als Wirkstoff eine Verbindung von Ansprüchen 1-3 enthalten.

5. Verwendung von Verbindungen der Formel I nach Anspruch 1 für die Herstellung eines antidepressiven Arzneimittels.

## Revendications

1. Composés de formule I : 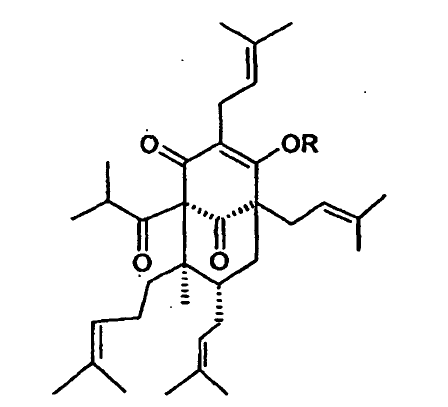 dans laquelle R est un résidu acyle aromatique dans lequel le fragment aromatique a facultativement un ou plusieurs substituants, qui peuvent être identiques ou différents, choisis parmi les groupes hydroxy, méthoxy, amino.

2. Composés selon la revendication 1, dans lesquels R est choisi parmi un groupe p-aminobenzoyle, triméthoxybenzoyle et triméthoxycinnamoyle.

3. Composé selon la revendication 2, dans lequel R est un groupe triméthoxybenzoyle.

4. Compositions pharmaceutiques contenant en tant qu'ingrédient actif un composé selon les revendications 1 à 3.

5. Utilisation des composés de formule 1 selon la revendication 1 pour la préparation d'un médicament antidépresseur.
